# EUROPEAN PATENT SPECIFICATION

(11) **EP 2 769 716 B1**
(45) Date of publication and mention of the grant of the patent: **16.11.2016**
(21) Application number: 12841268.1
(22) Date of filing: 19.10.2012
(51) Int. Cl.: A61K 31/138, A61K 31/135, A61P 9/00, A61P 25/28, A23L 2/00, A61K 9/08, A61K 9/14, A61K 9/16, A61K 9/20, A61K 9/48, A23L 2/52, A61K 9/00, A61K 31/137

(54) **PHARMACEUTICAL COMPOSITION COMPRISING FLUOXETINE AS AN ACTIVE INGREDIENT, FOR PREVENTING OR TREATING BLOOD-BRAIN BARRIER DISORDERS**
PHARMAZEUTISCHE ZUSAMMENSETZUNG MIT FLUOXETIN ALS WIRKSTOFF ZUR VORBEUGUNG ODER BEHANDLUNG VON ERKRANKUNGEN DER BLUT-HIRN-SCHRANKE
COMPOSITION PHARMACEUTIQUE COMPRENANT DE LA FLUOXÉTINE COMME INGRÉDIENT ACTIF, POUR PRÉVENIR OU TRAITER DES TROUBLES DE LA BARRIÈRE HÉMATO-ENCÉPHALIQUE

(30) Priority: 19.10.2011 KR 20110106982
(43) Date of publication of application: 27.08.2014
(73) Proprietor: University-Industry Cooperation Group of Kyung Hee University, Gyeonggi-do 446-701 (KR)
(72) Inventor: YUNE, Tae Young, Seoul 131-140 (KR); OH, Tae Hwan, Seoul 131-140 (KR); BAIK, Hyung Hwan, Seoul 131-800 (KR); LEE, Jee Youn, Seoul 139-771 (KR)
(74) Representative: Office Freylinger
(86) International application number: PCT/KR2012/008614
(87) International publication number: WO 2013/058605

(56) References cited:
- JP-A- 2009 523 802
- KR-A- 20080 051 246
- XUE-QIN LI ET AL: "Fluoxetine inhibited extracellular matrix of pulmonary artery and inflammation of lungs in monocrotaline-treated rats", ACTA PHARMACOLOGICA SINICA, vol. 32, no. 2, 10 January 2011 (2011-01-10), pages 217-222, XP055153193, ISSN: 1671-4083, DOI: 10.1038/aps.2010.187
- FENGSHAN YU ET AL: "Induction of MMP-9 Expression and Endothelial Injury by Oxidative Stress after Spinal Cord Injury", JOURNAL OF NEUROTRAUMA, vol. 25, no. 3, 1 March 2008 (2008-03-01), pages 184-195, XP055153182, ISSN: 0897-7151, DOI: 10.1089/neu.2007.0438
- WEISS N ET AL: "The blood-brain barrier in brain homeostasis and neurological diseases", BIOCHIMICA ET BIOPHYSICA ACTA (BBA) - BIOMEMBRANES, ELSEVIER, AMSTERDAM, NL, vol. 1788, no. 4, 1 April 2009 (2009-04-01), pages 842-857, XP026081419, ISSN: 0005-2736, DOI: 10.1016/J.BBAMEM.2008.10.022 [retrieved on 2008-11-11]
- TAE KYUONG SHIN ET AL.: 'Fluoxetine and Sertraline Attenuate Postischemic Brain Injury in Mice' KOREAN JOURNAL OF PHYSIOLOGY & PHARMACEOLOGY vol. 13, no. 3, 30 June 2009, pages 257 - 263, XP055128645
- LUCIANA S. BRANCO-DE-ALMEIDA ET AL.: 'Fluoxetine inhibitors inflammatory response and bone loss in a rat model of ligature-induced periodontitis.' JOURNAL OF PERIODONTOLOGY. vol. 83, no. 5, 03 October 2011, pages 664 - 671, XP008170864
- CHEOL HONG PARK ET AL.: 'Matrix Metalloproteinase Inhibitors Attenuate Neuroinflammation Following Focal Cerebral Ischemia in Mice.' KOREAN JOURNAL OF PHYSIOLOGY & PHARMACEOLOGY. vol. 15, no. 2, 30 April 2011, pages 115 - 122, XP055128648
- F CHOLLET ET AL: 'Fluoxetine for motor recovery after acute ischaemic stroke (FLAME): a randomised placebo-controlled trial' THE LANCET, [Online] 10 February 2011, XP055153169 DOI: 10.1016/S1474- Retrieved from the Internet: <URL:http://ac.els-cdn.com/S147444221070314 8/1-s2.0-S1474442210703148-main.pdf?_tid=8c b9209c-6c05-11e4-8de4-00000aab0f6b&acdnat=1 415973362_4638e75e9244802e85649429f1fc2514> [retrieved on 2014-11-14]

### SEQUENCE LISTING

<110> University-Industry Cooperation Group of Kyung Hee University
<120> PHARMACEUTICAL COMPOSITION COMPRISING FLUOXETINE AS AN ACTIVE
   INGREDIENT, FOR PREVENTING OR TREATING BLOOD-BRAIN BARRIER DISORDERS
<130> P-WON-091/WOEP
<150> KR10-2011-0106982
   <151> 2011-10-19
<160> 18
<170> BiSSAP 1.2
<210> 1
   <211> 20
   <212> DNA
   <213> Artificial Sequence
<220>
   <221> source
   <222> 1..20
   <223> /mol_type="unassigned DNA" /note="MMP-2 sense" /organism="Artificial Sequence"
<400> 1
   accatcgccc atcatcaagt 20
<210> 2
   <211> 20
   <212> DNA
   <213> Artificial Sequence
<220>
   <221> source
   <222> 1..20
   <223> /mol_type="unassigned DNA" /note="MMP-2 antisense" /organism="Artificial Sequence"
<400> 2
   cgagcaaaag catcatccac 20
<210> 3
   <211> 20
   <212> DNA
   <213> Artificial Sequence
<220>
   <221> source
   <222> 1..20
   <223> /mol_type="unassigned DNA" /notes-9 sense" /organism="Artificial Sequence"
<400> 3
   aaaggtcgct cggatggtta 20
<210> 4
   <211> 21
   <212> DNA
   <213> Artificial Sequence
<220>
   <221> source
   <222> 1..21
   <223> /mol_type="unassigned DNA" /note="MMP-9 antisense" /organism="Artificial Sequence"
<400> 4
   aggattgtct actggagtcg a 21
<210> 5
   <211> 20
   <212> DNA
   <213> Artificial Sequence
<220>
   <221> source
   <222> 1..20
   <223> /mol_type="unassigned DNA" /note="MMP-12 sense" /organism="Artificial Sequence"
<400> 5
   gaaaccccca tccttgacaa 20
<210> 6
   <211> 25
   <212> DNA
   <213> Artificial Sequence
<220>
   <221> source
   <222> 1..25
   <223> /mol_type="unassigned DNA" /note="MMP-12 antisense" /organism="Artificial Sequence"
<400> 6
   ttccaccaga agaaccagtc tttaa 25
<210> 7
   <211> 21
   <212> DNA
   <213> Artificial Sequence
<220>
   <221> source
   <222> 1..21
   <223> /mol_type="unassigned DNA" /note="GAPDH sense" /organism="Artificial Sequence"
<400> 7
   aggtcatccc agagctgaac g 21
<210> 8
   <211> 22
   <212> DNA
   <213> Artificial Sequence
<220>
   <221> source
   <222> 1..22
   <223> /mol_type="unassigned DNA" /note="GAPDH antisense" /organism="Artificial Sequence"
<400> 8
   caccctgttg ctgtagccgt at 22
<210> 9
   <211> 22
   <212> DNA
   <213> Artificial Sequence
<220>
   <221> source
   <222> 1..22
   <223> /mol_type="unassigned DNA" /note="CXCL-1 sense" /organism="Artificial Sequence"
<400> 9
   ccgaagtcat agccacactc aa 22
<210> 10
   <211> 25
   <212> DNA
   <213> Artificial Sequence
<220>
   <221> source
   <222> 1..25
   <223> /mol_type="unassigned DNA" /note="CXCL-1 antisense" /organism="Artificial Sequence"
<400> 10
   gcagtctgtc ttctttctcc gttac 25
<210> 11
   <211> 20
   <212> DNA
   <213> Artificial Sequence
<220>
   <221> source
   <222> 1..20
   <223> /mol_type="unassigned DNA" /note="CXCL-2 sense" /organism="Artificial Sequence"
<400> 11
   atccagagct tgacggtgac 20
<210> 12
   <211> 20
   <212> DNA
   <213> Artificial Sequence
<220>
   <221> source
   <222> 1..20
   <223> /mol_type="unassigned DNA" /note="CXCL-2 antisense" /organism="Artificial Sequence"
<400> 12
   aggtacgatc caggcttcct 20
<210> 13
   <211> 20
   <212> DNA
   <213> Artificial Sequence
<220>
   <221> source
   <222> 1..20
   <223> /mol_type="unassigned DNA" /note="CCL2 sense" /organism="Artificial Sequence"
<400> 13
   tcagccagat gcagttaacg 20
<210> 14
   <211> 22
   <212> DNA
   <213> Artificial Sequence
<220>
   <221> source
   <222> 1..22
   <223> /mol_type="unassigned DNA" /note="CCL2 antisense" /organism="Artificial Sequence"
<400> 14
   gatcctcttg tagctctcca gc 22
<210> 15
   <211> 22
   <212> DNA
   <213> Artificial Sequence
<220>
   <221> source
   <222> 1..22
   <223> /mol_type="unassigned DNA" /note="CCL3 sense" /organism="Artificial Sequence"
<400> 15
   actgcctgct gcttctccta ca 22
<210> 16
   <211> 21
   <212> DNA
   <213> Artificial Sequence
<220>
   <221> source
   <222> 1..21
   <223> /mol_type="unassigned DNA" /note="CCL3 antisense" /organism="Artificial Sequence"
<400> 16
   aggaaaatga cacctggctg g 21
<210> 17
   <211> 22
   <212> DNA
   <213> Artificial Sequence
<220>
   <221> source
   <222> 1..22
   <223> /mol_type="unassigned DNA" /note="CCL4 sense" /organism="Artificial Sequence"
<400> 17
   tcccacttcc tgctgtttct ct 22
<210> 18
   <211> 21
   <212> DNA
   <213> Artificial Sequence
<220>
   <221> source
   <222> 1..21
   <223> /mol_type="unassigned DNA" /note="CCL4 antisense" /organism="Artificial Sequence"
<400> 18
   gaataccaca gctggcttgg a 21

## Description

### [Technical Field]

The present invention relates to a pharmaceutical composition comprising fluoxetine as an active ingredient for use in recovering motor function deteriorated by spinal cord injury.

### [Background Art]

Lesions can be developed in central nervous system (CNS) with many different reasons. One of the major causes of such CNS lesions is stroke. Stroke is caused when a part of the brain stops being circulated and thereby neurons on the region lose their functions. Stroke, which is also called cerebrovascular accident or stroke syndrome, is the general term including thrombotic stroke, embolic stroke, and hemorrhagic stroke. According to the recent paper, it was reported that the incidence of all types of stroke is more than 500,000 cases annually. Stroke causes death or disability. Combining all types of stroke, stroke is the third major cause of death and the first major cause of disability. Considering the recent trend, the number of stroke cases is expected to grow million cases annually until 2050. The direct (nursing and treatment) and indirect (loss of productivity) costs consumed by stroke are estimated to be $43.3 billion annually in USA. Generally, stroke is classified into hemorrhagic stroke and ischemic stroke. Acute ischemic stroke is induced by thrombosis and embolism, which takes 80% of all stroke cases.

The major cause of cerebral infarction is thrombus or embolus in cerebral artery resulted from arteriosclerosis or cardiac embolism caused by heart disease. Cerebral infraction, induced by the blockage of cerebral blood vessels, is divided into two groups: cerebral thrombosis and cerebral embolism. Cerebral thrombosis is developed from arteriosclerosis caused by hypertension, diabetes, and hyperlipidemia, which is characterized by hardness or thickness of arterial wall. In cerebral thrombosis patients, blood vessels become narrow and the inner wall of the blood vessel is easily damaged and not smooth, so that blood clogging or cloudiness is observed, indicating that blood circulation is significantly disturbed or even stopped so that supply of oxygen and nutrients into the brain cells is lack, resulting in brain dysfunction. In the case of cerebral embolism, blood circulation in the heart is not smooth because of such disease as valvular heart disease or atrial fibrillation, so that partial blood retention is observed in the heart. At this time, the clogged blood is coagulated to produce blood clot and when the blood clot is chipped off and block the cerebral blood vessels, then cerebral infraction is developed.

Alzheimer's disease (AD) is characterized by neuronal loss and senile plaque comprising amyloid-beta (Aβ) protein, the 39-43 amino acid peptide, originated from amyloid precursor protein. Aβ or Aβ peptide fragment was confirmed to have cytotoxicity by *in vivo* and *in vitro* studies, indicating that Aβ plays an important role in the development of AD (Butterfield et al., Free Radical Biology and Medicine, 2002, 32:1050-1060 ; Butterfield et al., Free Radical Biology and Medicine, 2007, 43:658-677). During the culture, Aβ directly induced neuronal cell death or predisposed neurons to excitotoxicity and oxidative stress. NMDA (N-methyl-D-aspartate receptor) receptor is functioning as a selective matrix for Aβ binding or a mediator for Aβ-induced glutamate excitotoxicity. NMDA receptor is a ligand-gate/voltage-sensitive cation channel, which is highly transparent to Ca²⁺. A broad increase of [Ca²⁺]ᵢ results directly in cell dysfunction, hysteria, or cell death. As proved in the previous report that neurotoxic effect of Aβ was inhibited by [5R.10S)-(+)-5-methyl-10,11-dihydro-5H-dibenzo(a,b)cyclohepten-5,10-imine maleate] (MK-801), the non-competitive NMDA receptor antagonist, Ca²⁺ inflow via NMDA receptor exposed on Aβ plays a critical role in Aβ-induced neurotoxicity. The generation of reactive oxygen species (ROS) is also involved in the development of degenerative brain disease. The involvement of oxidative stress as an activator in Aβ-mediated neuropathy has been supported by the recent papers saying that a broad molecular shape that disturbs neuronal homeostasis can usher in or accelerate neurodegeneration. However, the clinical advantage of a direct blocker of NMDA receptor antagonist and neuronal cell channel is in doubt because of insufficient medicinal effect or serious side effects.

There is a barrier in neural tissue to block a specific material coming from the blood vessel. Such functional barrier is called 'blood-brain barrier (BBB)'. In blood-brain barrier, brain capillary that can keep homeostasis of neural microenvironment is well differentiated. The structure of blood-brain barrier is designed to allow only a minimum mobility of inessential materials, which is realized by maintaining vascular permeability of neural capillary lower than that of other tissue. The structure of blood-brain barrier is composed of tight junction or zonula occuluden in capillary endothelial cells. Unlike in capillaries of other regions, there is no fenestra in endothelial cytoplasm and pinocytotic vesicle is also rare therein. Low molecular weight materials necessary for neuronal tissue are conjugated with a carrier on cell membrane to enter the brain through endothelium. Blood-brain barrier prevents non-specific inflow of ions or proteins or other materials from entering CNS. Such role as a barrier helps to protect neurons from harmful components supplied from blood but let necessary materials in. Most cases of brain damage including cerebral infraction and trauma are related to the destruction of blood-brain barrier which further causes secondary damage in neurons. Therefore, the study on the development mechanism and regulation of blood-brain barrier is very important to understand and to treat central nervous system disease. Since many kinds of drugs and metabolites cannot pass through blood-brain barrier, proper regulation of blood-brain barrier seems to be the most important part to send drugs to target regions in order to treat various brain diseases including dementia. Blood-brain barrier is damaged by many diseases such as inflammatory disease and tumor, etc, and blood-brain barrier is still not functioning about 2 ∼ 3 weeks after the injury. Based on such characteristics, it is possible to trace lesion by observing how far and how deep a tracer substance sent in blood can penetrate in the brain. However, studies about the regulation of blood-brain barrier have not made satisfactory progress, yet.

The development of blood-brain barrier can be classified into the following two stages: cerebral angiogenesis and blood-brain barrier differentiation. In the first stage of cerebral angiogenesis, cerebrovascular endothelial cells induced from penetrable blood vessels invade avascular neurectoderm to form neural blood vessels. In the second stage of blood-brain barrier differentiation, brain capillary is differentiated with astrocytes in the late embryonic stage and the early birth stage and grows gradually to blood-brain barrier which is non-permeable. The said two stages of blood-brain barrier development can be regulated by oxygen partial pressure that also plays an important role in the development of blood vessels in many tissues or organs (Risau, W., Nature, 386, 671-674, 1997; Maltepe, E. & Simon, M.C., J. Mol. Med., 76, 391-401, 1998; Lee, Y.M. et al., Dev. Dyn., 220, 175-186, 2001). Previous studies reported that hypoxic region disappears gradually in the cerebral cortex of a rat in the middle of development (Lee, Y.M. et al., Dev. Dyn., 220, 175-186, 2001). Particularly, astrocytes inhibit cerebral angiogenesis in the state of reoxygenation and endothelial cells play an important role in the formation of blood-brain barrier, according to the previous reports (Song, H.S. et al., Biochem. Biophys. Res. Commun, 290, 325-331, 2002).

To be functioning appropriately, neurons need elaborately controlled extracellular environment. This fundamental and well-established microenvironment is locally preserved by nursing the brain cells called astrocytes (or astroglia). To cope with the considerable and variable heterogeneity between extracellular compartments of blood and brain, CNS is distinguished and shielded from the general blood circulation by numbers of blood-CNS barriers which are blood-brain barrier (BBB), blood-CSF barrier, ependyma and glia limitans, blood-retinal barrier, blood-neuron barrier, and blood-spinal cord barrier. Blood-brain barrier is regarded as the most important blood-CNS barrier because it covers the area 1000 times bigger than other blood-CNS barriers do. Blood-brain barrier is also characterized by the unique compact endothelial cell layer covering capillaries in CNS. Astrocytes protect glialfoot in capillary to induce blood-brain barrier activity in the endothelial cells.

Particularly, blood-brain barrier controls the flow of ions (Na⁺, F, Ca²⁺), water, nutrients, metabolites, neurotransmitters (glutamate, tryptophane), plasma proteins (albumin, fibrinogen, immunoglobulin), xenobiotics (drugs) that flow in and out of immune system to cells and the brain. Capillary endothelial cells in the brain have unique, different characteristics from peripheral capillary endothelial cells. That is, they are characterized by narrow tight junction, no fenestra, low pinocytotic activity and continuous basement membrane. The narrow tight junction causes as high electric resistance as 1500 ∼ 2000 Ohm.cm². Endothelial cells have negative surface charge rejecting negatively charged compounds. They have lots of mitochondria and enzymes that can destroy the selective transportation system and the compound that can actively deliver nutrients and other compounds in to or out of the brain. In healthy and normal condition, blood-brain barrier blocks drugs or endogenous compounds not to flow in the brain, so cellular infiltration is less than in the peripheral organs. Normal endothelial cell layer provides the anti-platelet surface that prevents the attachment of platelets and leukocytes and the activation of random coagulation system. Highly differentiated cerebral capillary endothelial cells form an elaborate barrier that can isolate the brain from immune surveillance and allow only a few monocytes (for example, activated T-cells) to move in CNS. The weak expression of major histocompatibility complex antigen, a small number of antigen presenting cells in normal CNS, and highly devleoped lymphatic vasculature make the brain "immunoisolated" part.

Basic anatomy of blood-brain barrier is understood only as dynamic control system that is affected by peripheral (for example, cortisol, adrenalin) and local (for example, cytokine, chemokine) hormones. In addition to astrocytes, pericytes, neurons, and immune cells also affect the characteristics of blood-brain barrier. In the meantime, endothelial cells are involved in other processes such as coagulation, antigen presentation, and growth factor mediated basement membrane regulation, etc. Particularly, activated endothelial cells play an important role under the pathological conditions such as inflammation in the brain and cerebrum and angiogenesis in brain tumor, etc.

Generally, blood-brain barrier is considered as the organ that protects cerebral homeostasis. Therefore, dysfunction of blood-brain barrier is a critical factor in most brain disorder cases. WEISS N et al.: "The blood-brain barrier in brain homeostasis and neurological diseases", BIOCHIMICA ET BIOPHYSICA ACTA (BBA) - BIOMEMBRANES, ELSEVIER, AMSTERDAM, NL, vol. 1788, no. 4, 1 April 2009, pages 842-857. Followings are some examples:
i. Cerebral angioedema is resulted from the inflow of water and plasma proteins from blood vessel to the brain tissue induced by disease (inflammation). It is a major reason of mal-functioning of body or even death in various cases of stroke, brain infection, head trauma, brain tumor, and multiple sclerosis. Edema induces the expansion of the brain in hard skull. Accordingly, intracranial pressure increases, which can be resulted in cerebral hernia. Later on, the fundamental brain function such as breathing goes bad and if not treated, serious disorder, coma, and even death are caused.
ii. In the case of multiple sclerosis, activated autoreactive T-cells can pass through blood-brain barrier. In CNS, those T-cells induce myelin sheath targeted inflammation response, which further induces the destruction of blood-brain barrier. Autoantibodies and complements lie over the destructed blood-brain barrier, which causes demyelination. Then, the fragments of myelin sheath flow in peripherals through the destroyed blood-brain barrier and they activate more autoreactive T-cells therein, indicating the increase of autoantibody production.
iii. Once the balance between neurotransmitters and microions in extracellular fluid is lost, malfunction of neuronal signal transduction is caused and thus cognitive function impairment, neuropsychiatric disorder, or epileptic seizure is caused.
iv. Blood-brain barrier injury over all the blood streams, which means toxic proteins are not eliminated, leads to neurodegenerative disease like Alzheimer's disease, and prion disease such as Creutzfeldt-Jakob disease and BSE. The pathological accumulation of such protein causes neuronal cell death, and thereby causes cognitive function impairment. Therefore, the treatment of malfunctioned blood-brain barrier leads to the solution of brain disorder.

It was reported that fluoxetine increased the anticonvulsant effects of carbamazepine, phenytoin, and ameltolide, which was confirmed by maximal electroshock convulsion test in mouse (Leander, J.D. Fluoxetine, a selective serotonin-uptake inhibitor enhances the anticonvulsant effects of phenytoin, carbamzepine, and ameltolide (LY201116). Epilepsai 33:573-576, 1992). Since fluoxetine can increase the effect of an antiepileptic drug co-treated there, it is also useful for the treatment of depressed patients having epilepsy.

A number of documents discuss the use of fluoxetine for treating different medical conditions, e.g. LUCIANA S. BRANCO-DE-ALMEIDA et al.: "Fluoxetine inhibitors inflammatory response and bone loss in a rat model of ligature-induced periodontitis.", JOURNAL OF PERIODONTOLOGY, vol. 83, no. 5, 3 October 2011, pages 664-671; XUE-QIN LI et al.: "Fluoxetine inhibited extracellular matrix of pulmonary artery and inflammation of lungs in monocrotaline-treated rats", ACTA PHARMACOLOGICA SINICA, vol. 32, no. 2, 10 January 2011, pages 217-222; TAE KYUONG SHIN et al.: "Fluoxetine and Sertraline Attenuate Postischemic Brain Injury in Mice", KOREAN JOURNAL OF PHYSIOLOGY & PHARMACEOLOGY, vol. 13, no. 3, 30 June 2009 (2009-06-30), pages 257-263. F Chollet et al.: "Fluoxetine for motor recovery after acute ischaemic stroke (FLAME): a randomised placebo-controlled trial", 10 February 2011, describe a double-blind, placebo-controlled trial, in which patients from nine stroke centres in France who had ischaemic stroke and hemiplegia or hemiparesis, were treated with fluoxetine (20 mg once per day, orally) or placebo for 3 months starting 5-10 days after the onset of stroke. Recovery of motor function was assessed using Fugl-Meyer motor scale (FMMS) scores.

Thus, the present inventors studied on the role of fluoxetine playing in blood-brain barrier. As a result, the inventors confirmed that fluoxetine inhibited the expressions of matrix metalloproteinase(MMP)-2, MMP-9 and MMP-12, which are reported to be related to blood-brain barrier disruption and inflammatory reaction after spinal cord injury (CHEOL HONG PARK et al.: "Matrix Metalloproteinase Inhibitors Attenuate Neuroinflammation Following Focal Cerebral Ischemia in Mice.", KOREAN JOURNAL OF PHYSIOLOGY & PHARMACOLOGY, vol. 15, no. 2, 30 April 2011, pages 115-122; ENGSHAN YU et al.: "Induction of MMP-9 Expression and Endothelial Injury by Oxidative Stress after Spinal Cord Injury" ,JOURNAL OF NEUROTRAUMA, vol. 25, no. 3, 1 March 2008, pages 184-195), remarkably reduced MMP-2 and MMP-9 activation, inhibited the decomposition of ZO-1, the most representative tight junction protein, so as to maintain a tight junction between endothelial cells and to protect blood-brain barrier thereby, inhibited the increase in permeability of the blood-brain barrier, and inhibited the expressions of chemoattractants of blood cells, such as CXCL-1, CXCL-2, CCL-2, CCL-3 and CCL-4 so as to reduce inflow of blood into spinal cord and recover motor function deteriorated by spinal cord injury. Accordingly, the present inventors completed this invention by confirming that fluoxetine could be effectively used for the treatment of spinal cord injury, in particular in a treatment for recovering motor function deteriorated by spinal cord injury.

### [Disclosure]

### [Technical Problem]

It is an object of the present invention to provide a pharmaceutical composition or a health food for use in the treatment of spinal cord injury comprising fluoxetine as an active ingredient, in which fluoxetine is effective in inhibiting the activity of MMP (matrix metalloproteinase) and suppressing the disruption of blood-brain barrier in central nervous system diseases such spinal cord injury.

### [Technical Solution]

To achieve the above objects, the present invention provides a pharmaceutical composition comprising fluoxetine for use in the treatment for recovering motor function deteriorated by spinal cord injury.

The present invention also provides a health food comprising fluoxetine for use in recovering motor function deteriorated by spinal cord injury.

### [Advantageous Effect]

In this invention, it was confirmed that fluoxetine inhibited the expressions of matrix metalloproteinase(MMP)-2, MMP-9 and MMP-12, reported to be related to blood-brain barrier disruption and inflammatory reaction after spinal cord injury, and remarkably reduced MMP-2 and MMP-9 activation, maintained a tight junction between endothelial cells to protect blood-brain barrier, inhibited the increase in permeability of the blood-brain barrier, and inhibited the expressions of chemoattractants of blood cells, such as CXCL-1, CXCL-2, CCL-2, CCL-3 and CCL-4 so as to reduce inflow of blood into spinal cord and recover motor function deteriorated by spinal cord injury. Therefore, fluoxetine can be effectively used as an active ingredient of a composition for recovering motor function deteriorated by spinal cord injury.

### [Description of Drawings]

The application of the preferred embodiments of the present invention is best understood with reference to the accompanying drawings, wherein:
Figure 1 is a diagram illustrating the effect of fluoxetine on the expression of MMP (matrix metalloproteinase) after spinal cord injury.
   S: group without spinal cord injury; and
   Vehicle: group treated with saline after spinal cord injury.
Figure 2 is a diagram illustrating the effect of fluoxetine on the activation of MMP(matrix metalloproteinase)-2 and MMP-9 after spinal cord injury.
   Flu: fluoxetine;
   SCI: spinal cord injury;
   S or Sham: group without spinal cord injury; and
   Veh or Vehicle: group treated with saline after spinal cord injury.
Figure 3 is a diagram illustrating the effect of fluoxetine on the decomposition of tight junction protein (TJP) after spinal cord injury.
   S or Sham: group without spinal cord injury; and
   Vehicle: group treated with saline after spinal cord injury.
Figure 4 is a diagram illustrating the effect of fluoxetine on the permeability of blood brain barrier (BBB) after spinal cord injury.
   Sham: group without spinal cord injury; and
   Vehicle: group treated with saline after spinal cord injury.
Figure 5 is a diagram illustrating the effect of fluoxetine on the inflow of blood cells into spinal cord after spinal cord injury.
   Sham: group without spinal cord injury; and
   Vehicle: group treated with saline after spinal cord injury.
Figure 6 is a diagram illustrating the effect of fluoxetine on the expression of chemoattractant after spinal cord injury.
   Flu: fluoxetine;
   S: group without spinal cord injury; and
   Veh or Vehicle: group treated with saline after spinal cord injury.
Figure 7 is a diagram illustrating the effect of fluoxetine on the recovery of motor function after spinal cord injury.
   Vehicle: group treated with saline after spinal cord injury.

### [Best Mode]

Hereinafter, the present invention is described in detail.

The present invention provides a pharmaceutical composition for for use in the treatment for recovering motor function deteriorated by spinal cord injury, comprising fluoxetine as an active ingredient.

The said fluoxetine is represented by the formula of C₁₇H₁₈F₃NO, but not always limited thereto.

Fluoxetine inhibits the disruption of blood-brain barrier (BBB), suppresses the expressions or activations of MMP(matrix metalloproteinase)-2, MMP-9, and MMP-12, inhibits the decomposition of tight junction protein (JTP), and suppresses the increase in permeability of the blood-brain barrier. Fluoxetine also inhibits the expression of chemoattractant to reduce blood inflow into spinal cord and to recover motor function deteriorated by spinal cord injury.

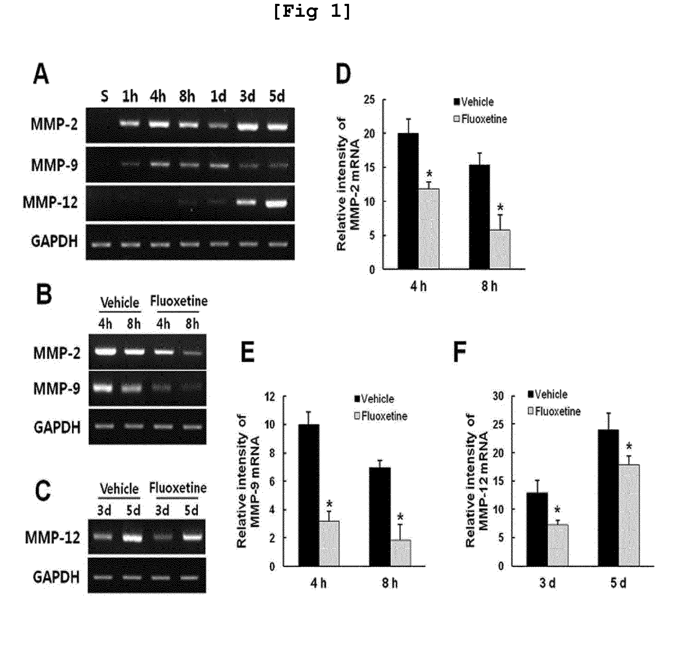

To investigate the inhibitory effect of fluoxetine on the expressions of MMP-2, MMP-9, and MMP-12, reported to be related to blood-brain barrier disruption and inflammatory reaction after spinal cord injury, the present inventors performed RT-PCR and observed the expressions of those genes. As a result, the expressions of MMP-2, MMP-9, and MMP-12 were all increased after spinal cord injury. Particularly, the expression of MMP-9 was increased most 4 hours ∼ 1 day after injury. The expression of MMP-12 was not observed until 1 day after injury, but from the third day, the expression was rapidly increased (see Figure 1A). In the meantime, the expressions of MMP-2, MMP-9, and MMP-12 were significantly reduced in the mouse treated with fluoxetine after spinal cord injury, compared with those in the mouse treated with saline (see Figure 1B ∼ Figure 1F).

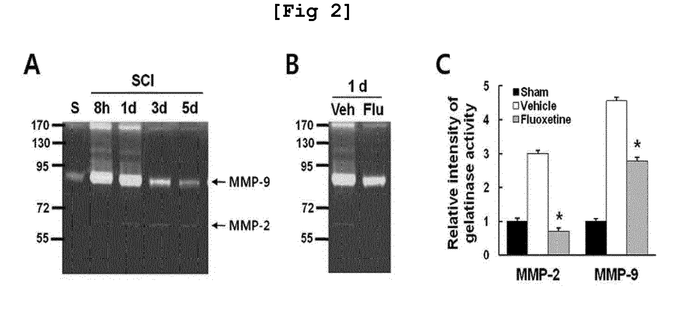

To investigate the inhibitory effect of fluoxetine on the activations of MMP-2 and MMP-9 after spinal cord injury, the present inventors measured the activation levels of MMP-2 and MMP-9 by using gelatin zymogram gel containing MMP-2/MMP-9 matrix, considering the characteristics of MMP that decomposes surrounding matrix when pro-form of the protein is digested and activated. As a result, a strong gelatinase activity was observed 8 hours after spinal cord injury, which began to decrease later on day 1 up to day 5. Comparison was performed between the mouse treated with saline and the mouse treated with fluoxetine 1 day (peak time) after spinal cord injury. As a result, MMP-2 and MMP-9 activities were significantly reduced in the mouse treated with fluoxetine after spinal cord injury (see Figure 2A ∼ Figure 2C).

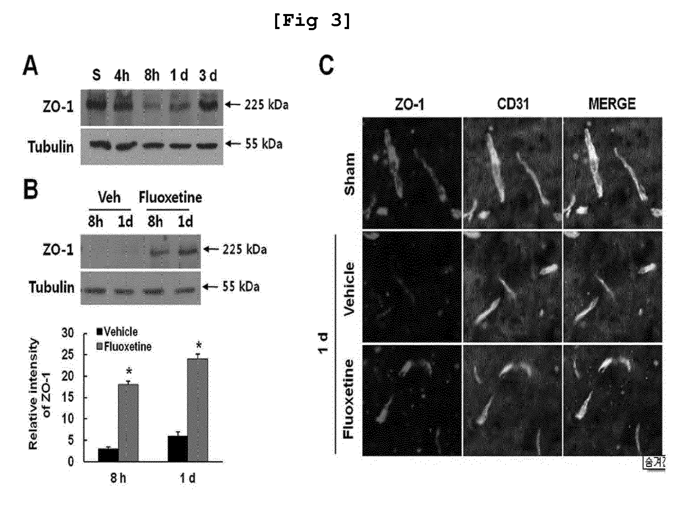

The decrease of tight junction protein (TJP) in endothelial cells forming blood vessel is one of major causes of blood-brain barrier destruction. Therefore, to investigate the inhibitory effect of fluoxetine on the decomposition of tight junction protein after spinal cord injury, the present inventors performed western blot analysis to measure the expression of ZO-1, the most representative tight junction protein. As a result, the amount of ZO-1 was significantly reduced 4 hours and 1 day after spinal cord injury (see Figure 3A). The amount of ZO-1 was not reduced in the mouse treated with fluoxetine after spinal cord injury, compared with that in the mouse treated with saline after spinal cord injury (see Figure 3B and Figure 3C). The above results indicate that fluoxetine inhibits the decomposition of ZO-1 after spinal cord injury, and rather maintains tight junction between endothelial cells to protect blood-brain barrier.

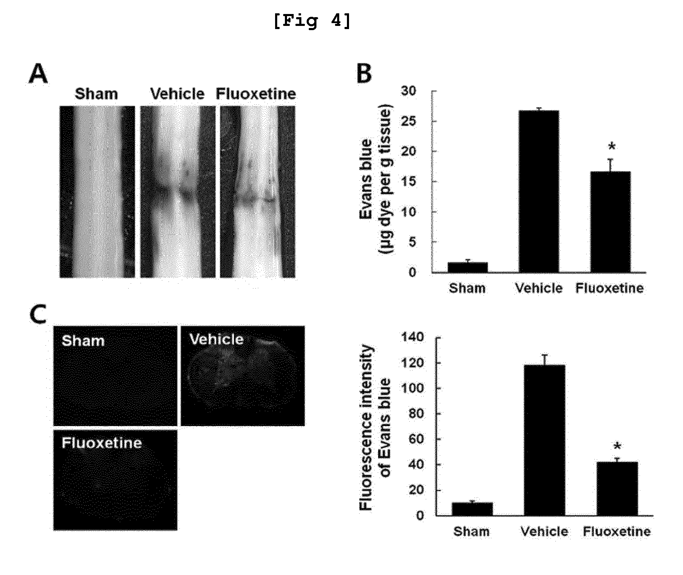

To investigate the inhibitory effect of fluoxetine on the increase of blood-brain barrier permeability after spinal cord injury, the present inventors performed following experiment. When blood-brain barrier permeability is increased according to spinal cord injury, blood is detected in spinal cord tissues, which is unusual in normal condition. Thus, blood-brain barrier permeability can be quantified by measuring the blood in spinal cord. Particularly, mice were treated with Evans Blue, the blue colloidal azodye that has high affinity to blood albumin, and then spinal cord was extracted three hours later. The amount of Evans Blue detected in spinal cord tissues was compared. As a result, the amount of Evans Blue was increased 25 times after spinal cord injury, which was reduced about 40% by fluoxetine treatment after spinal cord injury (see Figure 4A ∼ Figure 4C). These results indicate that fluoxetine inhibits blood-brain barrier permeability after spinal cord injury.

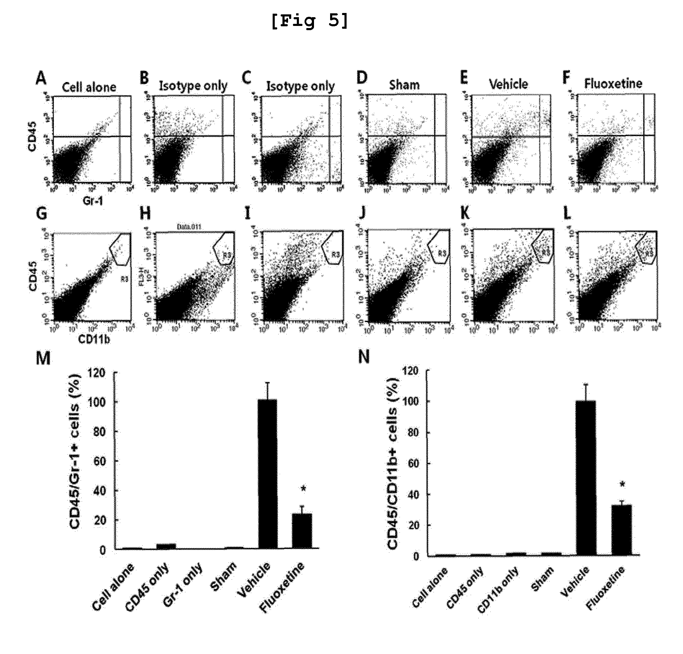

Once spinal cord is injured, various blood cells such as neutrophils, macrophages, and T-cells pass through the disrupted blood-brain barrier. 8 hours or a day after spinal cord injury, most of the infiltrated cells are neutrophils, but from the third day the infiltration of neutrophils decreases and the infiltration of macrophages increases instead. To investigate the inhibitory effect of fluoxetine on the inflow of blood cells into spinal cord after injury, the present inventors measured the amount of neutrophils and macrophages infiltrated on day 1 and day 5 by flow cytometry. The cells with high CD45 and Gr-1 were recognized as neutrophils, while the cells with high CD45 and CD11b were recognized as macrophages. As a result, the number of neutrophils and macrophages was significantly reduced in spinal cord tissues of the mouse treated with fluoxetine 1 and 5 days after spinal cord injury, compared with the mouse treated with saline after spinal cord injury (see Figure 5A ∼ Figure 5N).

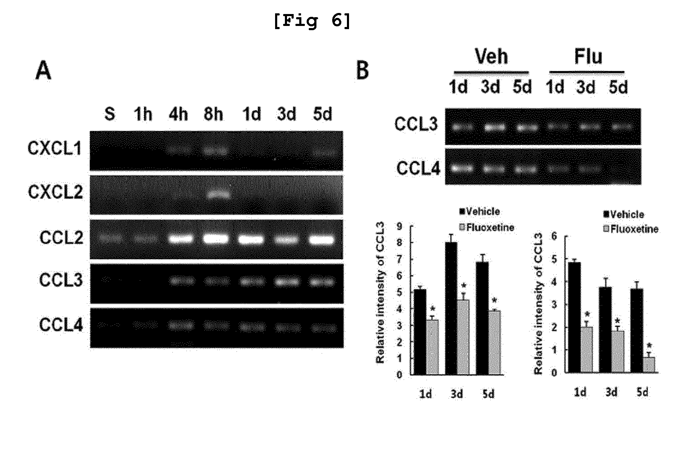

The present inventors also investigated whether or not the effect of fluoxetine reducing blood cells could result in regulation of the expression of chemoattractant inducing blood cell migration. Particularly, the present inventors performed RT-PCR to measure the expressions of CXCL-1, CXCL-2, CCL-2, CCL-3, and CCL-4 known as chemoattractants of neutrophils and monocytes. As a result, the expressions of such chemoattractants were significantly increased after spinal cord injury. Particularly, the expressions of CCL-3 and CCL-4 were suppressed by the treatment of fluoxetine 1 ∼ 5 days after spinal cord injury (see Figure 6A and Figure 6B). The above results indicate that fluoxetine inhibits the expression of chemoattractant of blood cells so as to reduce the inflow of blood into spinal cord.

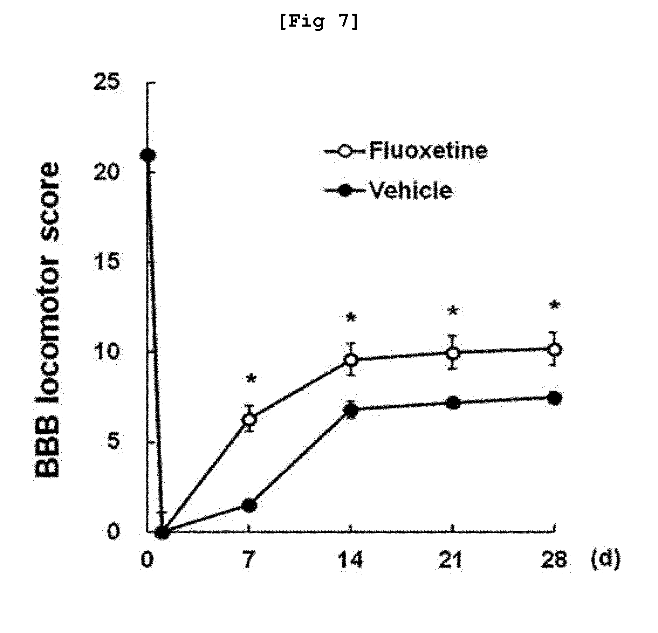

In addition, the present inventors also investigated the recovery of motor function by fluoxetine after spinal cord injury. To do so, the present inventors performed BBB test which facilitates the evaluation of recovery of various nerve fascicles controlling motor function in combination. As a result, after spinal cord injury, every mouse pulled its lower part only with forelegs without depending on hind legs which showed no movement at all. From 1 week after spinal cord injury, BBB points of the mouse treated with fluoxetine began to increase, compared with that of the mouse treated with saline only. BBB points were observed again 28 days after spinal cord injury. As a result, BBB point of the control mouse was 7.5±0.29 whose movement was limited in butt, knee, and ankle joint without supporting whole body weight. In the meantime, BBB point of the mouse treated with fluoxetine was 10.2±0.92 whose recovery could be as described as the mouse could walk with supporting its body weight with sole and the crossing frequency of forelegs and hind legs was approximately 50% and rather rhythmical during the walking, indicating significant recovery of motor function, compared with the control (see Figure 7).

Therefore, it was confirmed that the fluoxetine inhibited the expressions of MMP-2, MMP-9 and MMP-12, which are reported to be related to blood-brain barrier disruption and inflammatory reaction after spinal cord injury, and specifically reduced MMP-2 and MMP-9 activation, maintained tight junction between endothelial cells to protect blood-brain barrier, inhibited the increase in permeability of the blood-brain barrier, inhibited the expressions of chemoattractants of blood cells to reduce inflow of blood into spinal cord, and recovered motor function deteriorated by spinal cord injury. Therefore, the fluoxetine can be effectively used as an active ingredient of a composition for use in the treatment for recovering motor function deteriorated by spinal cord injury.

The composition of the present invention can additionally contain proper carriers, excipients, and diluents generally used for the preparation of a pharmaceutical composition.

The composition of the present invention can be administered orally or parenterally. The parenteral administration herein can be performed by external application to skin, intraperitoneal injection, intrarectal injection, hypodermic injection, intravenous injection, intramuscular injection, or intrathoracic injection, but not always limited thereto.

The composition of the present invention can be formulated for oral administration, for example powders, granules, tablets, capsules, suspensions, emulsions, syrups and aerosols, and for parenteral administration, for example external use, suppositories and sterile injections, etc. The carriers, exipients and diluents are exemplified by lactose, dextrose, sucrose, sorbitol, mannitol, xylitol, erythritol, maltitol, starch, acacia rubber, alginate, gelatin, calcium phosphate, calcium silcate, cellulose, methyl cellulose, microcrystalline cellulose, polyvinyl pyrrolidone, water, methylhydroxybenzoate, propylhydroxybenzoate, talc, magnesium stearate and mineral oil. Formulations can be prepared by using generally used excipients or diluents such as fillers, extenders, binders, wetting agents, disintegrating agents and surfactant. Solid formulations for oral administration are tablets, pills, powders, granules and capsules. These solid formulations are prepared by mixing one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized preparations, suppositories and injections. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The effective dosage of the composition of the present invention can be determined according to weight and condition of a patient, severity of a disease, preparation of a drug, administration pathway and time. The effective dosage of the composition of the present invention is preferably 0.0001 ∼ 1 g/kg per day, and more preferably 0.001 ∼ 200 mg/kg per day, but not always limited thereto. The administration frequency can be once a day or a few times a day. The above dosage cannot limit the scope of the invention in any way.

The present invention also provides a health food comprising fluoxetine for use in recovering motor function deteriorated by spinal cord injury.

The said fluoxetine is represented by the formula of C₁₇H₁₈F₃NO, but not always limited thereto.

Fluoxetine inhibits the disruption of blood-brain barrier (BBB), suppresses the expressions or activations of MMP(matrix metalloproteinase)-2, MMP-9, and MMP-12, inhibits the decomposition of tight junction protein (JTP), and suppresses the increase in permeability of the blood-brain barrier. Fluoxetine also inhibits the expression of chemoattractant to reduce blood inflow into spinal cord and to recover motor function deteriorated by spinal cord injury, but not always limited thereto.

The fluoxetine inhibited the expressions of MMP-2, MMP-9 and MMP-12, which are reported to be related to blood-brain barrier disruption and inflammatory reaction after spinal cord injury, and specifically reduced MMP-2 and MMP-9 activation, maintained tight junction between endothelial cells to protect blood-brain barrier, inhibited the increase in permeability of the blood-brain barrier, inhibited the expressions of chemoattractants of blood cells to reduce inflow of blood into spinal cord, and recovered motor function deteriorated by spinal cord injury. Therefore, fluoxetine can be effectively used as an active ingredient of a health food for the prevention and improvement of central nervous system disease or blood-brain barrier disorder.

The said fluoxetine can be used as a food additive for the preparation of the health food of the present invention. In that case, the fluoxetine can be added as it is or as mixed with other food components according to the conventional method.

The food herein is not limited. For example, the fluoxetine can be added to meats, sausages, breads, chocolates, candies, snacks, cookies, pizza, ramyuns, flour products, gums, dairy products including ice cream, soups, beverages, tea, drinks, alcohol drinks and vitamin complex, etc, and in wide sense, almost every food applicable in the production of health food can be included.

When the fluoxetine is used for food or beverages, the fluoxetine can be added as it is or after being mixed with other food or ingredients, according to the conventional method. The mixing ratio of active ingredients can be regulated according to the purpose of use (prevention, or improvement). In general, the fluoxetine is preferably added to food or beverages by 0.01 ∼ 15 weight% for the total weight of the food or beverages, for example it is preferably added by 0.02 ∼ 5 g and more preferably 0.3 ∼ 1 g to 100 mℓ of the health beverage. However, if long term administration is required for health and hygiene or regulating health condition, the content can be lower than the above but higher content can be accepted as well since the fluoxetine has been proved to be very safe.

The health beverages containing the fluoxetine can additionally include various flavors or natural carbohydrates, etc, like other beverages. The natural carbohydrates above can be one of monosaccharides such as glucose and fructose; disaccharides such as maltose and sucrose; polysaccharides such as dextrin and cyclodextrin; and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent.

In addition to the ingredients mentioned above, the health food of the present invention can include a variety of nutrients, vitamins, minerals (electrolytes), flavors including natural flavors and synthetic flavors, coloring agents and extenders (cheese, chocolate, etc.), pectic acid and its salts, alginic acid and its salts, organic acid, protective colloidal viscosifiers, pH regulators, stabilizers, antiseptics, glycerin, alcohols, carbonators which used to be added to soda, etc. The fluoxetine can also include natural fruit juice, fruit beverages and/or fruit flesh addable to vegetable beverages. All the mentioned ingredients can be added singly or together. The mixing ratio of those ingredients does not matter in fact, but in general, each can be added by 0 ∼ 20 weight part per 100 weight part of the fluoxetine of the present invention.

### [Mode for Invention]

Practical and presently preferred embodiments of the present invention are illustrative as shown in the following Examples.

### Example 1: Inhibition of MMP(matrix metalloproteinase) expression by fluoxetine after spinal cord injury <1-1> Construction of spinal cord injury animal model

Adult male C57BL/6 mice (20 ∼ 30 g) were anaesthetized with chloral hydrate (500 mg/kg) and T8 ∼ T10 were exposed. The exposed T9 was subjected to contusion injury (by dropping a weight at the impact strength of 40 kd) using PSI (Precision Systems and Instrumentation) impactor. After confirming that a certain damage was given within the error range based on the data of injury level, the incision sites were sealed. After disinfecting the incision sites, the mice were raised in cages (2 mice/cage). Urination was induced by massaging the bladder three times a day.

### <1-2> Fluoxetine treatment

Fluoxetine (Sigma, St. Louis, MO) dissolved in saline was immediately administered into injured mice via intraperitoneal injection (200 mg/kg). Saline was administered for the control. The administration was performed once a day and continued for 2 weeks.

### <1-3> Preparation of spinal cord tissue sections

Perfusion was performed through the right atrium in the mouse with spinal cord injury, followed by fixation in 4% parafomaldehyde. Spinal nerve was cut, followed by embedding in OCT compound, which was sectioned by using a cryostat. The spinal cord tissue for the extraction of RNA and protein proceeded to perfusion with PBS. Then, the spinal cord tissue was cut 0.8 cm from the injured area, which was stored in liquid nitrogen at -80°C until use.

### <1-4> RNA extraction and confirmation of MMP expression level by RT-PCR

RNA was extracted by using TRIZOL Reagent (Invitrogen) according to manufacturer's manual. Total reaction mixture (20 µl) containing 1 µg of RNA was prepared, followed by RT-PCR using MMLV reverse transcriptase (Invitrogen). MMP-2, MMP-9, and MMP-12 genes were amplified by using a template and each primer, followed by electrophoresis on agarose gel. The primers used for RT-PCR are as follows.

**[Table 1]**

| | | |
|---|---|---|
| MMP-2 | sense | 5'-ACC ATC GCC CAT CAT CAA GT-3' (SEQ. ID. NO: 1) |
| | antisense | 5'-CGA GCA AAA GCA TCA TCC AC-3' (SEQ. ID. NO: 2) |
| MMP-9 | sense | 5'- AAA GGT CGC TCG GAT GGT TA-3' (SEQ. ID. NO: 3) |
| | antisense | 5'-AGG ATT GTC TAC TGG AGT CGA-3' (SEQ. ID. NO: 4) |
| MMP-12 | sense | 5'-GAA ACC CCC ATC CTT GAC AA-3' (SEQ. ID. NO: 5) |
| | antisense | 5'-TTC CAC CAG AAG AAC CAG TCT TTA A-3' (SEQ. ID. NO: 6) |
| GAPDH | sense | 5'-AGG TCA TCC CAG AGC TGA ACG-3' (SEQ. ID. NO: 7) |
| | antisense | 5'-CAC CCT GTT GCT GTA GCC GTA T-3' (SEQ. ID. NO: 8) |

As a result, as shown in Figure 1A, the expressions of MMP-2, MMP-9, and MMP-12 were all increased after spinal cord injury. Particularly, the expression of MMP-9 was increased most 4 hours ∼ 1 day after injury. The expression of MMP-12 was not observed until 1 day after injury, but from the third day, the expression was rapidly increased (Figure 1A). In the meantime, the expressions of MMP-2, MMP-9, and MMP-12 were significantly reduced in the mouse treated with fluoxetine after spinal cord injury, compared with those in the mouse treated with saline after spinal cord injury (Figure 1B ∼ Figure IF).

### Example 2: Inhibition of MMP-2 and MMP-9 activation by fluoxetine after spinal cord injury

### <2-1> Inhibition of MMP-2 and MMP-9 activation confirmed by Zymography

Intracellular protein was obtained from the spinal cord tissue by using lysis buffer (1% Nonidet P-40, 50 mM Tris (pH 8.0), 150 mM NaCl, 0.5% sodium deoxycholate, 0.1% SDS). 30 ug of the obtained protein was loaded on Novex 10% gelatin zymogram gel (EC61752; Invitrogen), followed by electrophoresis at 4°C, 100 V. Then, the gel was loaded in renaturing buffer (2.5 % Triton X-100), followed by incubation at room temperature for 30 minutes. The gel was loaded in developing buffer (50 mM Tris-HCl, pH 8.5, 0.2 M NaCl, 5 mM CaCl₂, 0.02% Brii35), which stood at 37°C for 24 hours. After staining with Coomassie blue for 24 hours, the gel was placed in destaining buffer containing 40% methanol and 10% acetic acid. At this time, the clear band on the zymogram gel indicated gelatinase activity.

As a result, as shown in Figure 2A ∼ Figure 2C, a strong gelatinase activity was observed 8 hours after spinal cord injury, which began to decrease after 24 hours until day 5. The mouse treated with saline and the mouse treated with fluoxetine 1 day (peak time) after spinal cord injury were compared. As a result, MMP-2 and MMP-9 activities were significantly reduced in the spinal cord tissue of the mouse treated with fluoxetine after spinal cord injury (Figure 2A ∼ Figure 2C).

### Example 3: Inhibition of tight junction protein decomposition by fluoxetine after spinal cord injury Confirmation of ZO-1 expression pattern by western blot

Intracellular protein was obtained by using lysis buffer (1% Nonidet P-40, 50 mM Tris (pH 8.0), 150 mM NaCl, 0.5% sodium deoxycholate, 0.1% SDS), followed by SDS-PAGE. The protein was transferred onto nitrocellulose membrane, followed by western blot according to the protocol described in each antibody data sheet.

As a result, as shown in Figure 3A, the amount of ZO-1 was significantly reduced 8 hours and 1 day after spinal cord injury (Figure 3A). As shown in Figure 3B, the amount of ZO-1 was not reduced in the mouse treated with fluoxetine after spinal cord injury, compared with that in the mouse treated with saline after spinal cord injury (Figure 3B).

### <3-2> Immunohistochemical staining

10 µm tissue sections were reacted under the condition proposed in each primary antibody manual, followed by color development by using secondary antibodies. For immunofluorescence staining, secondary antibodies of Jackson were used. For DAB color development, Vectastatin kit (Vector) was used.

As a result, as shown in Figure 3B, ZO-1 was hardly reduced in the mouse treated with fluoxetine, compared with the mouse treated with saline after spinal cord injury (Figure 3B).

### Example 4: Inhibition of the increase of blood-brain barrier permeability by fluoxetine after spinal cord injury

### <4-1> Measurement of blood-brain barrier disruption by using Evans blue

To measure the disruption of blood-brain barrier, blood-brain barrier permeability was measured by using Evans blue. Particularly, 0.5 ml of 2% Evans blue solution dissolved in saline was administered intraperitoneally. Three hours later, perfusion with PBS was performed. The spinal cord tissue (4 mm) was removed from near and around the injured area and homogenized in 50% trichloroacetic acid solution.

After homogenization, the samples were centrifuged at 10,000xg for 10 minutes. Then, the supernatants were collected and fluorescence was quantified at an excitation wavelength of 620 nm and an emission wavelength of 680 nm.

As a result, as shown in Figure 4A ∼ Figure 4C, the amount of Evans blue was increased 25 times higher after spinal cord injury, which was reduced about 40% by fluoxetine treatment after spinal cord injury (Figure 4A ∼Figure 4C).

### Example 5: Inhibition of blood cell infiltration into spinal cord by fluoxetine

### <5-1> Inhibitory effect of fluoxetine on blood cell infiltration into spinal cord confirmed by flow cytometry

The spinal cord tissue was extracted 1 and 5 days after spinal cord injury, which was added into PBS. The spinal cord tissue in PBS was mechanically disrupted with a dounce homogenizer, followed by centrifugation at 1,100 rpm for 10 minutes. The obtained pellet was resuspended in FBS containing buffer, which was centrifuged at 3,000 rpm for 7 minutes. The obtained pellet was resuspended in FBS containing buffer again, which was split into several tubes, followed by reaction with fluorescein-conjugated antibody at 4°C. After washing with FBS containing buffer, neutrophils and macrophages infiltrated into spinal cord 1 and 5 days after spinal cord injury were measured by using Becton Dickinson LSR Benchtop Flow Cytometer (BD Biosciences). The cells with high CD45 and Gr-1 were recognized as neutrophils, while the cells with high CD45 and CD11b were recognized as macrophages.

As a result, as shown in Figure 5A ∼ Figure 5N, the number of neutrophils and macrophages was significantly reduced in spinal cord tissues of the mouse treated with fluoxetine 1 and 5 days after spinal cord injury, compared with the mouse treated with saline after spinal cord injury (Figure 5A ∼ Figure 5N).

### Example 6: Inhibition of chemoattractant expression by fluoxetine after spinal cord injury

The expressions of blood cell chemoattractants, CXCL-1, CXCL-2, CCL-2, CCL-3, and CCL-4, were measured by the same manner as described in Example <1-4>. As a result, as shown in Figure 6A and Figure 6B, the expressions of such chemoattractants (chemokines) were significantly increased after spinal cord injury. Particularly, the expressions of CCL-3 and CCL-4 were suppressed by the treatment of fluoxetine 1 ∼ 5 days after spinal cord injury (Figure 6A and Figure 6B).

**[Table 2]**

| | | |
|---|---|---|
| CXCL-1 | sense | 5'-CCG AAG TCA TAG CCA CAC TCA A-3' (SEQ. ID. NO: 9) |
| | antisense | 5'-GCA GTC TGT CTT CTT TCT CCG TTA C-3' (SEQ. ID. NO: 10) |
| CXCL-2 | sense | 5'-ATC CAG AGC TTG ACG GTG AC-3' (SEQ. ID. NO: 11) |
| | antisense | 5'-AGG TAC GAT CCA GGC TTC CT-3' (SEQ. ID. NO: 12) |
| CCL2 | sense | 5'-TCA GCC AGA TGC AGT TAA CG-3' (SEQ. ID. NO: 13) |
| | antisense | 5'-GAT CCT CTT GTA GCT CTC CAG C-3' (SEQ. ID. NO: 14) |
| CCL3 | sense | 5'-ACT GCC TGC TGC TTC TCC TAC A-3' (SEQ. ID. NO: 15) |
| | antisense | 5'-AGG AAA ATG ACA CCT GGC TGG-3' (SEQ. ID. NO: 16) |
| CCL4 | sense | 5'-TCC CAC TTC CTG CTG TTT CTC T-3' (SEQ. ID. NO: 17) |
| | antisense | 5'-GAA TAC CAC AGC TGG CTT GGA-3' (SEQ. ID. NO: 18) |

### Example 7: Effect of fluoxetine on the recovery of motor function after spinal cord injury

### <7-1> Animal behavioral test (BBB test)

BBB test is the method to evaluate the recovery of various nerve fascicles controlling motor function in combination, in which scores are recorded from 0 to 21. Particularly, when the hind legs did not move at all, the score was given 0. A normal mouse got 21 scores for that. So, the motor function is regarded to be improved as the score got higher. Mice were placed in a plastic open field box (90 cm x 90 cm x 7 cm), once a day, 4 minutes per mouse at a time, from 3 days before spinal cord injury. The mice were let go freely in the box so as for the mice to be adapted to the experimental environment. From the next day after spinal cord injury, 5 trained experimenters observed the movement of the hind legs of the mice and gave BBB scores accordingly, twice a week. The average of BBB test scores obtained from those five trained experimenters was considered as the final BBB score for the mouse. To eliminate personal prejudice on the test animal, the experimenters were not given any information on the mice. They only observed the mice 4 minutes every time to give scores.

As a result, as shown in Figure 7, every mouse pulled its lower part only with forelegs without depending on hind legs which showed no movement at all after spinal cord injury. From 1 week after spinal cord injury, BBB points of the mouse treated with fluoxetine began to increase, compared with that of the mouse treated with saline only. BBB points were observed again 28 days after spinal cord injury. As a result, BBB point of the control mouse was 7.5±0.29 whose movement was limited in butt, knee, and ankle joint without supporting whole body weight. In the meantime, BBB point of the mouse treated with fluoxetine was 10.2±0.92 whose recovery could be as described as the mouse could walk with supporting its body weight with sole and the crossing frequency of forelegs and hind legs was approximately 50% and rather rhythmical during the walking, indicating significant recovery of motor function, compared with the control (Figure 7).

The Manufacturing Examples for the composition of the present invention are described hereinafter.

### Manufacturing Example 1: Preparation of pharmaceutical formulations containing fluoxetine

### <1-1> Preparation of powders

| | |
|---|---|
| Fluoxetine | 2 g |
| Lactose | 1 g |

Powders were prepared by mixing all the above components, which were filled in airtight packs according to the conventional method for preparing powders.

### <1-2> preparation of tablets

| | |
|---|---|
| Fluoxetine | 100 mg |
| Corn starch | 100 mg |
| Lactose | 100 mg |
| Magnesium stearate | 2 mg |

Tablets were prepared by mixing all the above components by the conventional method for preparing tablets.

### <1-3> Preparation of capsules

| | |
|---|---|
| Fluoxetine | 100 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

### <1-4> Preparation of pills

| | |
|---|---|
| Fluoxetine | 1 g |
| Lactose | 1.5 g |
| Glycerin | 1 g |
| Xylitol | 0.5 g |

Pills were prepared by mixing all the above components according to the conventional method for preparing pills. Each pill contained 4 g of the mixture.

### <1-5> Preparation of granules

| | |
|---|---|
| Fluoxetine | 150 mg |
| Soybean extract | 50 mg |
| Glucose | 200 mg |
| Starch | 600 mg |

All the above components were mixed, to which 100 mg of 30% ethanol was added. The mixture was dried at 60°C and the prepared granules were filled in packs.

### Manufacturing Example 2: Preparation of health foods containing fluoxetine

Health foods containing the fluoxetine were prepared as follows.

### <2-1> Preparation of flour food

0.5 ∼ 5.0 weight part of the fluoxetine was added to the flour. Health enhancing foods such as bread, cake, cookies, crackers and noodles were prepared with the flour mixture according to the conventional method.

### <2-2> Preparation of soups and gravies

0.1 ∼ 5.0 weight part of the fluoxetine was added to soups and gravies. Health enhancing meat products, soups and gravies were prepared with this mixture by the conventional method.

### <2-3> Preparation of ground beef

Health enhancing ground beef was prepared by mixing 10 weight part of the fluoxetine with ground beef according to the conventional method.

### <2-4> Preparation of dairy products

5 ∼ 10 weight part of the fluoxetine was added to milk. Health enhancing dairy products such as butter and ice cream were prepared with the milk mixture according to the conventional method.

### <2-5> Preparation of Sun-Sik

Brown rice, barley, glutinous rice and Yulmu (Job's tears) were gelatinized according to the conventional method, dried and pulverized to obtain 60-mesh powders.

Black soybean, black sesame and wild sesame were steamed and dried according to the conventional method and pulverized to obtain 60-mesh powders.

The fluoxetine was concentrated under reduced pressure, spray-dried and pulverized to obtain 60-mesh dry powders.

Sun-Sik was prepared by mixing the dry powders of the grains, seeds and the fluoxetine according to the below ratio.
Grains (brown rice: 30 weight part, Yulmu: 15 weight part, barley: 20 weight part),
Seeds (wild sesame: 7 weight part, black soybean: 8 weight part, black sesame: 7 weight part),
Dry powders of the fluoxetine (3 weight part), *Ganoderma lucidum* (0.5 weight part),
*Rehmannia glutinosa* (0.5 weight part).

### Manufacturing Example 3: Preparation of beverages containing fluoxetine

### <3-1> Preparation of health beverages

5 g of the fluoxetine was mixed with liquid fructose (0.5%), oligosaccharide (2%), sugar (2%), salt (0.5%), and water (75%). After mixing completely, the mixture was sterilized instantly and filled small containers such as glass bottles, pet bottles, etc, to prepare health beverages.

### <3-2> Preparation of vegetable juice

Health enhancing vegetable juice was prepared by adding 5 g of the fluoxetine to 1,000 mℓ of tomato or carrot juice according to the conventional method.

### <3-3> Preparation of fruit juice

Health enhancing fruit juice was prepared by adding 1 g of the fluoxetine to 1,000 mℓ of apple or grape juice according to the conventional method.

### [Industrial Applicability]

As explained hereinbefore, fluoxetine inhibits the destruction of blood-brain barrier (BBB), so that it can be effectively used for the development of a drug or a health food for use in recovering motor function deteriorated by spinal cord injury.

### <2-3> Preparation of ground beef

Health enhancing ground beef was prepared by mixing 10 weight part of the fluoxetine of the present invention with ground beef according to the conventional method.

### <2-4> Preparation of dairy products

5 ∼ 10 weight part of the fluoxetine of the present invention was added to milk. Health enhancing dairy products such as butter and ice cream were prepared with the milk mixture according to the conventional method.

### <2-5> Preparation of Sun-Sik

Brown rice, barley, glutinous rice and Yulmu (Job's tears) were gelatinized according to the conventional method, dried and pulverized to obtain 60-mesh powders.

Black soybean, black sesame and wild sesame were steamed and dried according to the conventional method and pulverized to obtain 60-mesh powders.

The fluoxetine of the present invention was concentrated under reduced pressure, spray-dried and pulverized to obtain 60-mesh dry powders.

Sun-Sik was prepared by mixing the dry powders of the grains, seeds and the fluoxetine of the present invention according to the below ratio.
Grains (brown rice: 30 weight part, Yulmu: 15 weight part, barley: 20 weight part),
Seeds (wild sesame: 7 weight part, black soybean: 8 weight part, black sesame: 7 weight part),
Dry powders of the fluoxetine of the present invention (3 weight part),
*Ganoderma lucidum* (0.5 weight part),
*Rehmannia glutinosa* (0.5 weight part).

### Manufacturing Example 3: Preparation of beverages containing fluoxetine

### <3-1> Preparation of health beverages

5 g of the fluoxetine of the present invention was mixed with liquid fructose (0.5%), oligosaccharide (2%), sugar (2%), salt (0.5%), and water (75%). After mixing completely, the mixture was sterilized instantly and filled small containers such as glass bottles, pet bottles, etc, to prepare health beverages.

### <3-2> Preparation of vegetable juice

Health enhancing vegetable juice was prepared by adding 5 g of the fluoxetine of the present invention to 1,000 mℓ of tomato or carrot juice according to the conventional method.

### <3-3> Preparation of fruit juice

Health enhancing fruit juice was prepared by adding 1 g of the fluoxetine of the present invention to 1,000 mℓ of apple or grape juice according to the conventional method.

### [Industrial Applicability]

As explained hereinbefore, the fluoxetine of the present invention inhibits the destruction of blood-brain barrier (BBB), so that it can be effectively used for the development of a drug or a health food to prevent, improve, or treat central nervous system disease or blood-brain barrier disorder, or for a study to develop a method for the prevention or treatment of those diseases using the same.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

## Claims

1. A pharmaceutical composition comprising fluoxetine for use in the treatment for recovering motor function deteriorated by spinal cord injury.

2. The pharmaceutical composition for use of claim 1, wherein the composition further comprises a pharmaceutically acceptable carrier, an excipient or a diluent.

3. The pharmaceutical composition for use of claim 1, wherein the composition is in a form selected from the group consisting of injectable solution, tablet, pill, powder, granule and capsule.

4. A health food comprising fluoxetine for use in recovering motor function deteriorated by spinal cord injury.

5. The health food comprising fluoxetine for use of claim 4, wherein the composition further comprises a food additive selected from the group consisting of a nutrient, a vitamin, a mineral, a flavoring agent, a coloring agent, a pectic acid or salt thereof, an alginic acid or salt thereof, an organic acid, a protective colloidal viscosifier, a pH regulator, a stabilizer, an antiseptic, a glycerin, an alcohols and a carbonator.

6. The health food comprising fluoxetine for use of claim 4 or 5, wherein the composition is in a form selected from the group consisting of meat, sausage, bread, chocolate, candy, snack, cookie, pizza, ramyun, flour product, gum, dairy product, soup, a beverage, a tea, a drink, an alcoholic beverage, a vitamin complex, a fruit and a vegetable juice.

## Patentansprüche

1. Pharmazeutische Zusammensetzung, die Fluoxetin umfasst, zur Verwendung bei der Behandlung zur Wiederherstellung der Motorik, die sich durch eine Rückenmarksverletzung verschlechtert hat.

2. Pharmazeutische Zusammensetzung zur Verwendung nach Anspruch 1, wobei die Zusammensetzung weiterhin einen pharmazeutisch akzeptablen Trägerstoff, einen Hilfsstoff oder ein Verdünnungsmittel umfasst.

3. Pharmazeutische Zusammensetzung zur Verwendung nach Anspruch 1, wobei die Zusammensetzung in einer Form ist, die aus der Gruppe bestehend aus einer Injektionslösung, einer Tablette, einer Pille, einem Pulver, einem Granulat und einer Kapsel ausgewählt ist.

4. Reformkost, die Fluoxetin umfasst, zur Verwendung bei der Wiederherstellung der Motorik, die sich durch eine Rückenmarksverletzung verschlechtert hat.

5. Reformkost, die Fluoxetin umfasst, zur Verwendung nach Anspruch 4, wobei die Zusammensetzung weiterhin einen Nahrungsmittelzusatzstoff umfasst, der aus der Gruppe bestehend aus einem Nährstoff, einem Vitamin, einem Mineral, einem Aromastoff, einem Farbstoff, einer Pektinsäure oder einem Salz davon, einer Alginsäure oder einem Salz davon, einer organischen Säure, einem Schutzkolloid-Eindickmittel, einem Mittel zur Regulierung des pH-Werts, einem Stabilisierungsmittel, einem Antiseptikum, einem Glycerin, einem Alkohol und einem Karbonisierungsmittel ausgewählt ist.

6. Reformkost, die Fluoxetin umfasst, zur Verwendung nach Anspruch 4 oder 5, wobei die Zusammensetzung in einer Form ist, die aus der Gruppe bestehend aus Fleisch, Wurst, Brot, Schokolade, Süssigkeit, Snack, Keks, Pizza, Instant-Nudeln, Mehlprodukt, Kaugummi, Milchprodukt, Suppe, einem Getränk, einem Tee, einem Trunk, einem alkoholischen Getränk, einem Vitaminkomplex, einem Obst- und einem Gemüsesaft ausgewählt ist.

## Revendications

1. Composition pharmaceutique comprenant de la fluoxétine pour une utilisation dans le traitement pour la récupération de la fonction motrice détériorée par une lésion de la moelle épinière.

2. Composition pharmaceutique pour une utilisation selon la revendication 1, dans laquelle la composition comprend en outre un support, un excipient ou un diluant pharmaceutiquement acceptable.

3. Composition pharmaceutique pour une utilisation selon la revendication 1, dans laquelle la composition est sous une forme choisie dans le groupe constitué par une solution injectable, un comprimé, une pilule, une poudre, un granulé et une gélule.

4. Aliment de santé comprenant de la fluoxétine pour une utilisation dans la récupération de la fonction motrice détériorée par une lésion de la moelle épinière.

5. Aliment de santé, comprenant de la fluoxétine pour une utilisation selon la revendication 4, dans lequel la composition comprend en outre un additif alimentaire choisi dans le groupe constitué par un nutriment, une vitamine, un minéral, un agent aromatisant, un agent colorant, un acide pectique ou un sel de celui-ci, un acide alginique ou un sel de celui-ci, un acide organique, un agent viscosifiant colloïdal protecteur, un régulateur de pH, un stabilisant, un antiseptique, une glycérine, un alcool et un agent de carbonatation.

6. Aliment de santé comprenant de la fluoxétine pour une utilisation selon la revendication 4 ou 5, dans lequel la composition est sous une forme choisie dans le groupe constitué par la viande, une saucisse, le pain, le chocolat, les bonbons, une collation, un biscuit, une pizza, des nouilles instantanées (« ramyun »), un produit de farine, une gomme, un produit laitier, une soupe, un breuvage, un thé, une boisson, un breuvage alcoolisé, un complexe vitaminique, un jus de fruit et un jus de légume.
